# EUROPEAN PATENT SPECIFICATION

(11) **EP 0 998 296 B1**
(45) Date of publication and mention of the grant of the patent: **10.07.2002**
(21) Application number: 98933828.0
(22) Date of filing: 24.07.1998
(51) Int. Cl.: A61K 35/78, A61P 3/06

(54) **PHARMACEUTICAL USES OF CYPRESS**
PHARMAZEUTISCHE VERWENDUNGEN VON ZYPRESSEN
UTILISATIONS PHARMACEUTIQUES DU CYPRES

(30) Priority: 24.07.1997 GR 97100293; 22.04.1998 GR 98100141
(43) Date of publication of application: 10.05.2000
(73) Proprietor: Zografos-Dietetic Products S.A.C.I., 185 40 Piraeus (GR)
(72) Inventor: ZOGRAFOS, Nikolaos, GR-185 40 Piraeus (GR)
(86) International application number: GR9800022
(87) International publication number: WO9907398

(56) References cited:
- WO-A-90/13304
- FR-A- 2 184 550
- FR-M- 1 834

## Description

Hypercholesterolemia and hyperlipidemia of dietary and congenital origin are metabolic disturbances (disorders), which are encountered at an increased frequency among the human population, due to living conditions (nutrition, stress) and bear severe negative long term consequences on health.

The treatment attempted, preventively and therapeutically, is at present based on a combination of diet, exercise and administration of various types of pharmacologically active substances. There are two main types of pharmacologically active substances: These substances which interfere in the process of absorption of cholesterol and triglycerides from the gastrointestinals and those substances which interfere by inhibiting the endogenous metabolic way of cholesterol synthesis or promote its catabolism.

The alcoholic extract of the fruits of the coniferous Cupressus Sempervirens was prepared in order to be used for the prevention and treatment of the metabolic disturbances of cholesterol and lipids in general, in humans and animals.

An alcoholic extract of the fruits of the cypress tree has been prepared, using 100 ml of absolut alcohol for every 10 fruits of the cypress. Three weeks are required for the extraction prossess to be completed.

The alcoholic extract has been at first administered in experimental animals (Wistar rats) subject to a diet rich in cholesterol. The experiment was conducted on two groups of rats: The first group was fed only with the rich in cholesterol diet (control group), whereas the second group was, simultaneously, fed with the rich in cholesterol (hypercholesterolemic) diet and administered (per os) with 10 drops of the alcoholic extract, per day, for 3 months.

Each group comprised of 10 animals.

At the end of the trimester, animals from both groups were sacrificed and blood levels of cholesterol, LDL, HDL and triglycerides were measured. Furthermore, autopsy of the aorta and all vital organs was also performed. From the measurements it was found that rats, from the group that was given the extract, exhibited a reduction of cholesterol levels by 33±5%, an increase of HDL levels by 22±3% and a reduction of LDL levels by 38±7%, with respect to rats from the control group that was only fed with the rich in cholesterol diet. Furthermore, it was found that animals of the control group that did not take the extract, exhibited atheromatic lesions of the aorta, whereas rats of the group fed with the hypercholesterolemic (rich in cholesterol) diet and administered by the alcoholic extract, at the same time, did not exhibit any such lesions.

None of the vital organs of the animals administered by the extract manifested any lesion in autopsy in contrast to the control group, where fat infiltration of liver was obvious.

Then, the extract was administered at daily doses of 90 drops (divided in three portions, 30 drops x 3), in seven hypercholesterolemic volunteers, per os, without any diet restrictions for 2 months. After the first 15 days of administration, a statistically significant decrease of cholesterol and LDL levels was observed with a parallel increase of HDL levels. Biochemical tests (SGOT/SGPT, alkaline phosphatase, urea, creatinine, uric acid, γ-GT) as well as routine blood tests did not reveal any other changes to volunteers condition before and after the administration of the alcoholic extract.

Furthermore, the sublimation of the alcoholic extract was tried: The solid residue obtained from the sublimation of the extract has the form of a brownish powder. This powder was administered into two groups of rats suffering from dietary-induced hyperlipidemia, as follows:
1st group (7 animals): The powder was administered (per os, daily, for three months) as a suspension in drinking water. The dosage is the same as the powder contained in one millilitre of the extract.
2nd group (7 animals): A proportional to the 1st group amount of powder per day was administered (for three months) as an oily preparation with the food pellets provided to the experimental animals.

A statistically significant decrease of cholesterol and LDL levels was found in both groups in comparison to the levels before administration ( p<0,001 ). A slight increase of HDL levels was also found ( p<0,045 ) in comparison to the levels recorded before administration.

Then, the powder was administered at doses of 3 mg per day (divided in three portions, 1 mg x 3), in 5 hypercholesterolemic volunteers, per os, without any dietetic restrictions for 2 months. After the first 15 days of administration, a statistically significant decrease of cholesterol and LDL levels was observed in parallel with an increase of HDL levels. General biochemical examination (SGOT/SGPT, alkaline phosphatase, urea, creatinine, uric acid, γ-GT) as well as routine blood tests of these volunteers did not reveal any differences than the values before administration.

From the above, it was potently evidenced that substances contained in the fruits of Cupressus Sempervirens, exert a preventive and probably therapeutic effect on dietary-induced hyperlipidemia in humans and animals.

Experiments conducted so far have given data indicating that similar effects are exerted by products that are isolated by other types of elaborations (oily and aqueous extracts, condensates of the alcoholic extracts, powders from the sublimation of alcoholic extracts, active constituents of alcoholic and oily extracts) from the fruits as well as other parts (roots, trunk, cortex, leaves e.t.c.) from Cupressus Sempervirens as well as other species of the genus Cupressus.

From the alcoholic extract of the fruits of the coniferous Cupressus Sempervirens, were isolated [ by condensation, evaporation, high pressure liquid chromatography (HPLC), gas chromatography (GC) and thin layer chromatography (TLC) ] nine different fractions of active, mainly polar compounds belonging to the groups of terpenoids, polyphenols and proanthocyanidins. By the use of Mass Spectroscopy, it was found that the main constituents of these fractions are the α-cedrol (60% of the extract) and manool (12%).

A mixture of the isolated fractions of the above mentioned groups of substances, as well as the α-cedrol and manool and a mixture of the latter substances (in analogy of 6:1) were administered in four groups of experimental animals (Wistar rats) suffering from dietary-induced hypercholesterolemia. Each group comprised of 10 rats. The substances were daily administered per os (orally), for 2 months, as a solution and the daily dose (in mg/Kg body weight) was calculated.

Blood samples were taken from the rats for determination of cholesterol, HDL and LDL, before the administration, after 15 and 30 days from the beginning of the administration of the fractions, as well as at the end of 2 months of administration.

From the results the following were found:
1) 15 days of administration resulted in a statistically significant decrease of cholesterol and LDL levels (about 15±3,2%) in all four groups (group of fractions' combination, group of α-cedrol, group, of manool and group of cedrol and manool combination) in comparison to the control group. The lowest effect, was observed in the group of manool.
2) At the end of the first and second month of administration, the reduction of cholesterol and LDL was remarkably higher (about 30±5% in comparison to the control group). The lowest effect was observed in the group of manool.
3) The HDL did not manifest any significant alteration in all four groups. Blood and biochemical tests revealed no pathological effects.
4) From autopsy (in comparison to the control group which suffered from dietary-induced hypercholesterolemia), a significant reduction of aortic atheromatic lesions was found in the rats of all four groups of animals.

In addition, synthetic α-cedrol was administered (at similar doses to the naturally isolated α-cedrol) in groups of dietary-induced hypercholesterolemic rats with similar (to the naturally isolated α-cedrol) results.

Following, the combination (mixture) of the isolated fractions as well as natural and synthetic α-cedrol and manool were tested as inhibitors of platelet aggregation (induced by ADP, collagen and thrombin) in washed rabbit platelets and platelet rich plasma of healthy volunteers. It was found that the fractions' mixture as well as the previously mentioned substances and their combinations exert a high anti-aggregatory effect on the three agonists (ADP, collagen and thrombin) with more potent the effect of the mixture.

By the, up today, experimental tests, there are indications that the above mentioned mixture as well as the above mentioned substances and their combinations, exert a protective and probably therapeutic effect on hypercholesterolemia and hyperlipidemia of dietary or congenital origin, in humans and animals.

The protective effect of these substances may be attributed to: a) the significant reduction of cholesterol and LDL levels, b) the inhibition of platelet aggregation, c) the possible protection from LDL oxidation. That is, the protection exhibited by these substances may be attributed to their actions against the processes which are considered to be significant factors of atheromatosis.

There were also made preparations containing the above mentioned products as well as the fractions' mixtures and the above mentioned substances and their combinations or the esters or salts of these substances. These preparations are pharmaceutical preparations (administered per os or parenterally or by any other route) as well as preparations used as additives in any type of food or drink.

Experimental tests conducted so far have given indications that the above preparations exert a protective and possibly therapeutic effect on hypercholesterolemia and hyperlipidemia of dietary or congenital origin, in humans and animals.

The experimental application of the above products, the fractions' mixtures, the substances and the above mentioned preparations, has not given any evidence of toxic effects or adverse reactions, even at significantly higher dosages than those refered above and for a remarkably longer period of time, of almost six months of administration.

## Claims

1. Use of products (alcoholic extract, condensate of the alcoholic extract, powder and active constituents of the alcoholic extract) which are isolated by every type of elaboration of parts from the group of fruit, cortex, leaves, root, and trunk of cupressus sempervirens and all other species of the genus Cupressus, for the manufacture of a medicament or for preparing an additive, in every type of food or drink, for the prevention and treatment of any type of hypercholesterolemia and lipids' disturbances, in humans and animals.

2. Use of products, according to claim 1, which specifically are the alcoholic extracts of parts from the group of fruit, cortex, leaves, root, and trunk of cupressus sempervirens, for the manufacture of a medicament or for preparing an additive, in every type of food or drink, for the prevention and treatment of any type of hypercholesterolemia and lipids' disturbances, in humans and animals.

3. Use of product, according to claims 1 and 2, which is specifically the alcoholic extract of the fruits of cupressus sempervirens, for the manufacture of a medicament or for preparing an additive, in every type of food or drink, for the prevention and treatment of any type of hypercholesterolemia and lipids' disturbances, in humans and animals.

4. Use of product, according to claim 3, which is specifically a condensate of the alcoholic extract of cupressus' fruits, for the manufacture of a medicament or for preparing an additive, in every type of food or drink, for the prevention and treatment of any type of hypercholesterolemia and lipids' disturbances, in humans and animals.

5. Use of product, according to claim 3, which is specifically the solid residue (in powder form) from the sublimation of the alcoholic extract of cupressus' fruits, for the manufacture of a medicament or for preparing an additive, in every type of food or drink, for the prevention and treatment of any type of hypercholesterolemia and lipids' disturbances, in humans and animals.

6. Use of products, according to claim 3, that specifically are the active constituents which are isolated from the alcoholic extract of cupressus' fruits, for the manufacture of a medicament or for preparing an additive, in every type of food or drink, for the prevention and treatment of any type of hypercholesterolemia and lipids' disturbances, in humans and animals.

7. Use of substances, according to claims 1 and 6, derived from the alcoholic extract of the fruits of cupressus sempervirens and belonging to the groups of terpenoids having as characteristic representatives the cedrols, polyphenols and proanthocyanidins, administered either single or in combinations (mixtures), exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

8. Use of substances, according to claim 7, which specifically belong to the group of the terpenoid having as characteristic representative the cedrols, exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

9. Use of substances, according to daim 7, which specifically belong to the group of polyphenols, exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

10. Use of substances, according to claim 7, which specifically belong to the group of the proanthocyanidins, exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

11. Use of substances, according to claims 7 and 8, which specifically are cedrols having as characteristic representative the α-cedrol, exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

12. Use of mixtures of substances, according to claims 7, 8, 9 and 10, which specifically are combinations of terpenoids, polyphenols and proanthocyanidins, exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

13. Use of mixtures of substances, according to claims 7, 8 and 9, which specifically are combinations of terpenoids and polyphenols, exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

14. Use of substances and mixtures of substances, which belong to the groups of terpenoids having as characteristic representatives the cedrols, polyphenols and proanthocyanidins, which are of synthetic origin (synthetically manufactured), exerting anticholesterolemic and antiplatelet (inhibition of platelet aggregation) effects, for the manufacture of a medicament or for preparing an additive, in every type of food or drink, for the prevention and treatment of any type of hypercholesterolemia and lipids' disturbances, in humans and animals.

15. Use of products, substances and mixtures of them, mentioned in claims 1 to 14, for the manufacture of a medicament which is a pharmaceutical product, administrable per os or parenterally or by any other route, for the prevention and treatment of any type of hypercholesterolemia and lipids' disturbances, in humans and animals.

16. Use of products, substances and mixtures of them, mentioned in claims 1 to 14, for preparing additives used in every type of food or drink, for the prevention and treatment of any type of hypercholesterolemia and lipids' disturbances, in humans and animals.

## Patentansprüche

1. Anwendung von Produkten - alkoholisches Extrakt, Konzentrat des alkoholischen Extrakts, Pulver und Wirkstoffe des alkoholischen Extrakts - die durch die Verarbeitung der Bestandteile, aus den Früchten, Schalen, Wurzeln und Stamm des cupressus seprevirens und aller weiteren Cypressus - Arten zur Herstellung eines Medikaments, oder zur Herstellung eines Zusatzmittels für alle Getränke- und Lebensmittelarten, zur Vorbeugung allerlei Hypercholesterinämie- und Lipidenstörungen bei Menschen und Tieren gewonnen werden.

2. Anwendung von Produkten gemäß Anforderung 1, die insbesondere die alkoholischen Extrakte der Bestandteile aus den Früchten, Schale, Wurzel und Stamm des cupressus seprevirens und aller weiteren Cypressus - Arten zur Herstellung eines Medikaments, oder zur Herstellung eines Zusatzmittels für alle Getränke- und Lebensmittelarten, zur Vorbeugung und Behandlung (Therapie) allerlei Hypercholesterinämie- und Lipidenstörungen bei Menschen und Tieren, sind.

3. Anwendung von Produkten gemäß Anforderungen 1 & 2, die insbesondere das alkoholische Extrakt der Bestandteile aus den Früchten des cupressus seprevirens zur Herstellung eines Medikaments, oder zur Herstellung eines Zusatzmittels in allen Getränke- und Lebensmittelarten, zur Vorbeugung und Behandlung (Therapie) von allerlei Hypercholesterinämie und Lipidenstörungen bei Menschen und Tiere, sind.

4. Anwendung von einem Produkt gemäß Anforderung 3, das insbesondere das Konzentrat des aus den Früchten des cupressus seprevirens abstammenden alkoholischen Extrakts zur Herstellung eines Medikaments, oder zur Herstellung eines Zusatzmittels in allen Getränke- und Lebensmittelarten, zur Vorbeugung und Behandlung (Therapie) von allerlei HypercholesterinÄmie und Lipidenstörungen bei Menschen und Tieren ist.

5. Anwendung von einem Produkt gemäß Anforderung 3, das insbesondere dar aus der Verdampfung des Extraktes aus den Früchten des cupressus seprevirens abstammende Feststoffrest - in Pulverform - zur Herstellung des Medikaments, oder zur Herstellung eines Zusatzmittels in allen Getränkeund Lebensmittelarten, zur Vorbeugung und Behandlung (Therapie) allerlei Hypercholesterinämie und Lipidenstörungen bei Menschen und Tieren ist.

6. Anwendung von Produkten gemäß Anforderung 3, welche die Wirkstoffe, die aus dem alkoholischen Extrakt der Früchte der Zupresse cupressus seprevirens zur Herstellung eines Medikaments, oder eines Zusatzmittels in allen Getränke- und Lebensmittelarten, zur Vorbeugung und Behandlung (Therapie) allerlei Hypercholesterinämie und Lipidenstörungen bei Menschen und Tieren gewonnen werden, sind.

7. Anwendung von Substanzen, gemäß Anforderungen 1 und 6, die aus dem alkoholischen Extrakt der Früchten der Zypresse cupressus seprevirens gewonnen werden, und den Terpenoiden gehören, deren Vertreter die Zedrolen, Polyphänole und Proantho Cyanidinen sind, die entweder alleine, oder in Mischungen, die Cholesterol- und Thrombozytenhemmende - Hemmung der Ansammlung von Thrombozyten - Wirkung aufweisen, verabreicht werden, zur Herstellung eines Medikaments, oder zur Herstellung eines Zusatzmittels, zur Vorbeugung und Behandlung von allerlei Hypercholesterinämie und Lipidenstörungen bei Menschen und Tieren.

8. Anwendung von Substanzen, gemäß Anforderung 7, die den Terpenoiden gehören, deren Vertreter die Zedrolen, die Anti-Cholesterol- und antiaimopetaliaki - Hemmung der Ansammlung von Thrombozyten - Wirkung aufweisen, zur Herstellung eines Medikaments, oder zur Herstellung eines Zusatzmittels zur Vorbeugung und Behandlung von allerlei Hypercholesterinämie- und Lipidenstörungen bei Menschen und Tieren.

9. Anwendung von Substanzen, gemäß Anforderung 7, die den Polyphänolen gehören, die eine cholesterol- und thrombozytenhemmende Wirkung - Hemmung der Ansammlung von Thrombozyten - aufweisen, zur Herstellung eines Medikaments, oder zur Herstellung eines Zusatzmittels, zur Vorbeugung und Behandlung von allerlei Hypercholesterinämie- und Lipidenstörungen bei Menschen und Tieren.

10. Anwendung von Substanzen, gemäß Anforderung 7, die den Proantho Cyanidine gehören, die eine cholesterol- und thrombozytenhemmende Wirkung - Hemmung der Ansammlung von Thrombozyten - aufweisen, verabreicht werden, zur Herstellung eines Medikaments, oder zur Herstellung eines Zusatzmittels, zur Vorbeugung und Behandlung von allerlei Hypercholesterinämie- und Lipidenstörung bei Menschen und Tieren.

11. Anwendung von Substanzen, gemäß Anforderungen 7 und 8, die insbesondere die Zedrolen sind, deren Vertreter die a-Zedrol ist, die eine cholesterol- und thrombozytenhemmende Wirkung - Hemmung der Ansammlung von Thrombozyten - aufweisen, zur Herstellung eines Medikaments, oder zur Herstellung eines Zusatzmittels, zur Vorbeugung und Behandlung von allerlei Hypercholesterinämie- und Lipidenstörung bei Menschen und Tieren.

12. Anwendung von Substanzen, gemäß Anforderungen 7, 8, 9 und 10, die Kombinationen von Terpenoiden, Polyphänolen und Proantho Cyanidinen darstellen, und eine cholesterol- und thrombozytenhemmende Wirkung - Hemmung der Ansammlung von Thrombozyten - aufweisen, zur Herstellung eines Medikaments, oder zur Herstellung eines Zusatzmittels, zur Vorbeugung und Behandlung von allerlei Hypercholesterinämie- und Lipidenstörung bei Menschen und Tieren.

13. Anwendung von Mischungen, gemäß Anforderungen 7, 8 und 9, welche insbesondere Kombinationen von Terpenoiden und Polyphänolen darstellen, und eine cholesterol- und thrombozytenhemmende Wirkung - Hemmung der Ansammlung von Thrombozyten - aufweisen, zur Herstellung eines Medikaments, oder zur Herstellung eines Zusatzmittels, zur Vorbeugung und Behandlung von allerlei Hypercholesterinämie- und Lipidenstörungen bei Menschen und Tieren.

14. Anwendung von Substanzen und Mischungen von Substanzen, die den Terpenoiden angehören, deren Vertreter die Zedrolen, die Polyphänole und Proantho Cyanidinen sind, die synthetisch (künstlich) hergestellt werden und eine cholesterol- und thrombozytenhemmende Wirkung - Hemmung der Ansammlung von Thrombozyten - aufweisen, zur Herstellung eines Medikaments, oder zur Herstellung eines Zusatzmittsl, zur Vorbeugung und Behandlung von allerlei Hypercholesterinämie- und Lipidenstörungen bei Menschen und Tieren.

15. Anwendung der Produkte, Substanzen und Mischungen, die in den Anforderungen 1 bis 14 aufgeführt werden, zur Herstellung eines durch den Mund, oder den Darm o.a. verabreichten Medikaments (pharmazeutischen Produktes), zur Vorbeugung und Behandlung von allerlei Hypercholesterinämie- und Lipidenstörungen bei Menschen und Tieren.

16. Anwendung der Produkte, der Substanzen und deren Mischungen, die in den Anforderungen 1 bis 14 aufgeführt werden, zur Herstellung von Zusatzstoffen, die bei jeder Lebensmittel- oder Getränkart, zur Vorbeugung und Behandlung von allerlei Hypercholesterinämie- und Lipidenstörungen bei Menschen und Tieren, eingesetzt werden.

## Revendications

1. Utilisation des produits -extrait alcoolique, concentré de l'extrait alcoolique, poudre et éléments actifs de l'extrait alcoolique- qui sont isolés après tout type de traitement des parties, du groupe des fruits, de l'écorce, de la racine et du tronc, du cyprès cupressus sempervirens et de toutes autres espèces du genre Cupressus, pour la préparation d'un médicament ou d'un additif pour toutes les sortes d'aliment ou de boisson, pour la prévention et le traitement de tous types d'hypercholéstélolemie et de désordre lipidique, aux êtres humains et aux animaux.

2. Utilisation des produits, selon la revendication 1, qui sont en particuliers des extraits alcooliques des parties -du groupe des fruits, de l'écorce, des feuilles, de la racine et du tronc, du cyprès cupressus sempervirens, pour la préparation d'un médicament ou d'un additif pour toutes les sortes d'aliment ou de boisson, pour la prévention et le traitement de tous types d'hypercholéstélolemie et de désordre lipidique, aux êtres humains et aux animaux.

3. Utilisation d'un produit, selon les revendications 1 et 2, qui est en particuliers l'extrait alcoolique des fruits du cyprès cupressus sempervirens, pour la préparation d'un médicament ou d'un additif pour toutes les sores d'aliment ou de boisson, pour la prévention et le traitement de tous types d'hypercholéstélolemie et de désordre lipidique, aux êtres humains et aux animaux.

4. Utilisation d'un produit, selon la revendication 3, qui est en particuliers l'extrait alcoolique des fruits du cyprès cupressus sempervirens, pour la préparation d'un médicament ou d'un additif pour toutes les sortes d'aliment ou de boisson, pour la prévention et le traitement de tous types d'hypercholéstélolemie et de désordre lipidique, aux êtres humains et aux animaux.

5. Utilisation d'un produit, selon la revendication 3, qui est en particuliers le résidu solide -en forme de poudre- de la sublimation de l'extrait alcoolique des fruits du cyprès cupressus sempervirens, pour la préparation d'un médicament ou d'un additif pour tous les types d'aliment ou de boisson, pour la prévention et le traitement de tous types d'hypercholéstélolemie et de désordre lipidique, aux êtres humains et aux animaux.

6. Utilisation des produits, selon la revendication 3, qui sont en particuliers les éléments actifs isolés de l'extrait alcoolique des fruits du cyprès cupressus sempervirens pour la préparation d'un médicament ou d'un additif pour toutes les sortes d'aliment ou de boisson, pour la prévention et le traitement de tous types d'hypercholéstélolemie et de désordre lipidique, aux êtres humains et aux animaux.

7. Utilisation des substances, selon les revendications 1 et 6, qui se produisent par l'extrait alcoolique des fruits du cyprès cupressus sempervirens et appartiennent au groupes des terpenoides, ayant comme représentants typiques les cedroles, de polyphénols et de proanthocyanides, qui s'administrent soit seules soit en combinaison -mixtions- et ont une action anticholéstélolemique et antithrombocytique -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

8. Utilisation des substances, selon la revendication 7, qui, en particuliers, appartiennent au groupe des terpenoides, ayant comme représentants typiques les cedroles, qui ont une action anticholéstélolemique et antithrombocytique -inhibition d'accumulation des plaquettes- pour la préparation d'un médicament ou d'un additif pour tous les types d'aliment ou de boisson, pour la prévention et le traitement de tous types d'hypercholéstérolemie et de désordre lipidique, aux êtres humains et aux animaux.

9. Utilisation des substances, selon la revendication 7, qui, en particuliers, appartiennent au groupe des polyphénols et ont une action anticholéstélolemique et antithrombocytique -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

10. Utilisation des substances, selon la revendication 7, qui, en particuliers, appartiennent au groupe des proanthocyanides et ont une action anticholéstélolemique et antithrombocytique -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

11. Utilisation des substances, selon les revendications 7 et 8, qui, en particuliers, sont de cedroles, ayant comme représentant typique la cedrole-a, qui ont une action anticholéstélolemique et antithrombocytique -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

12. Utilisation des mixtions de substances, selon les revendications 7, 8, 9 et 10, qui sont, en particuliers, des combinaisons des terpenoides, polyphénols et proanthocyanides et ont une action anticholéstélolemique et antithrombocytiques -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

13. Utilisation des mixtions de substances, selon les revendications 7, 8 et 9, qui sont, en particuliers, des combinaisons des terpenoides et polyphénols et ont une action anticholéstélolemique et antithrombocytique -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

14. Utilisation de substances et des mixtions de substances, qui appartiennent aux groupes de terpenoides, ayant comme représentants typiques les cedroles, de polyphénols et de proanthocyanides, d'origine synthetique (produites par synthèse), qui ont une action anticholéstélolemique et antithrombocytique -inhibition d'accumulation des plaquettes- pour la préparation d'un médicament ou d'un additif pour toutes les sortes d'aliment ou de boisson, pour la prévention et le traitement de tous types d'hypercholéstérolemie et de désordre lipidique, aux êtres humains et aux animaux.

15. Utilisation de produits, substances et leurs mixtions de substances, qui sont mentionnés dans les revendications 1 jusqu'au 14 incluse, pour la préparation d'un médicament qui sera un produit pharmaceutique (pharmaceutical product), administré par la voie orale ou parenterique ou de quelconque autre voie, pour la prévention et le traitement de tous types d'hypercholéstérolemie et de désordre lipidique, aux êtres humains et aux animaux.

16. Utilisation de produits, substances et leurs mixtions de substances, qui sont mentionnés dans les revendications 1 jusqu'au 14 incluse, pour la préparation des additifs utilisés dans toutes sortes d'aliments ou de boisson, pour la prévention et le traitement de tous types d'hypercholéstérolemie et de désordre lipidique, aux êtres humains et aux animaux.
